# EUROPEAN PATENT SPECIFICATION

(11) **EP 2 115 021 B1**
(45) Date of publication and mention of the grant of the patent: **25.07.2012**
(21) Application number: 08709602.0
(22) Date of filing: 08.02.2008
(51) Int. Cl.: C08F 220/56, G01N 33/50

(54) **SOLID PHASE EXTRACTION OF AFLATOXINS**
FESTPHASENEXTRAKTION VON AFLATOXINEN
EXTRACTION EN PHASE SOLIDE D'AFLATOXINES

(30) Priority: 09.02.2007 GB 0702489
(43) Date of publication of application: 11.11.2009
(73) Proprietor: Toximet Limited, Kent Science Park Sittingbourne Kent ME9 8AZ (GB)
(72) Inventor: COKER, Raymond, Bromley Kent BR1 2PJ (GB); PILETSKY, Sergey, Cranfield Bedfordshire MK43 OEE (GB); PILETSKA, Olena, Bedfordhire MK43 0EE (GB)
(74) Representative: Brookes Batchellor LLP
(86) International application number: PCT/GB2008/050082
(87) International publication number: WO 2008/096179

(56) References cited:
- TURNER N W ET AL: "Effect of the solvent on recognition properties of molecularly imprinted polymer specific for ochratoxin A" BIOSENSORS & BIOELECTRONICS, ELSEVIER SCIENCE PUBLISHERS, BARKING, GB, vol. 20, no. 6, 15 December 2004 (2004-12-15), pages 1060-1067, XP004648746 ISSN: 0956-5663
- SAIRAM ET AL: "Encapsulation efficiency and controlled release characteristics of crosslinked polyacrylamide particles" INTERNATIONAL JOURNAL OF PHARMACEUTICS, AMSTERDAM, NL, vol. 320, no. 1-2, 31 August 2006 (2006-08-31), pages 131-136, XP005586487 ISSN: 0378-5173
- DATABASE BIOSIS BIOSCIENCES INFORMATION SERVICE, PHILADELPHIA, PA, US; 31 August 2006 (2006-08-31), M. SAIRA: "Encapsulation efficiency and controlled release characteristics of crosslinked polyacrylamide particles" XP002478279

## Description

### Field of the Invention

The present invention relates to a polymer suitable for binding of aflatoxins (B₁, B₂, G₁ & G₂). The polymer may be used for solid phase extraction of aflatoxins and immobilisation of aflatoxins in solid phase extraction (SPE) cartridges for qualitative or quantitative analysis of aflatoxins in solutions extracted from foodstuffs or feedstuffs.

### Background of the Invention

A wide variety of human foods and animal feeds, including edible nuts, oilseeds, cereal grains, and forages and products derived from them are susceptible to contamination by mycotoxins, which are toxic metabolic by-products of fungi which can occur on food and feed crops both before and after harvest. Among the most significant are the aflatoxins, a group of closely-related mycotoxins produced by the fungi *Aspergillus flavus* and *A. parasiticus.* Not all isolates of the fungi produce aflatoxins; thus the mere presence of *A. flavus* or A. parasiticus does not mean that aflatoxins will be present in the substrate. Accordingly direct determination of aflatoxin level is an important aspect of quality control in foods and feeds.

Such measurements have conventionally been carried out by the use of high performance liquid chromatography (HPLC). However in those cases where HPLC equipment is not available or appropriate, determination by thin layer chromatography (TLC) is also possible. Commercial scanners are available for mycotoxin determination after TLC separation, using mercury lamps with an emission wavelength of 366 nm as a light source to stimulate fluorescence, which is detected and quantified by photo-multipliers. For quantitative testing there are also radioimmunoassay techniques and immunochemically-based techniques such as enzyme-linked immunosorbent assay (ELISA) methods.

Before a solution obtained by extraction from a foodstuff sample is subjected to quantitative measurement, using HPLC for example, the solution may be subjected to a 'clean-up' procedure using solid phase extraction to remove compounds that may interfere with the mycotoxin evaluation.

Qualitative detection of mycotoxins can be carried out using small chromatographic columns (so-called 'mini-columns') in which the mycotoxins are immobilised as a layer within a mineral adsorbent in the mini-columns. The minicolumns are viewed under ultraviolet light to cause the immobilised mycotoxin to fluoresce. Various minicolumn methods have been adopted as official tests of the AOAC International (Association of Official Analytical Communities).

WO 2006/123189 describes apparatus for the quantitative fluorometric assay of mycotoxins immobilised in layers in minicolumns and also mentions use of molecularly imprinted polymer and non-molecularly imprinted (blank) polymers as absorbents for mycotoxins in SPE cartridges.

WO 2003/101580 discloses a method of binding mycotoxins to a solid carrier which includes preparing a molecularly imprinted polymer (MIP) using the mycotoxin as template. From the extensive lists of mycotoxins and functional monomers that are mentioned, the only MIPs actually prepared use the mycotoxins deoxynivalenol (DON) and zearalenone (ZON) as templates. The MIP for DON is polymerised from diethylaminoethyl-methacrylate (DEAMA), 4-vinylpyridine (4-VP) or methacrylic acid (MAA) with ethylenediglycol-methacrylate (EDMA) or divinylbenzene (DVP) as cross-linker. The MIP for ZON is polymerised from trifluoromethacrylic acid (TFM) or 4-VP using EDMA as cross-linker.

Turner, N.W et al. "Effect of the solvent recognition properties of molecularly imprinted polymer specific for ochratoxins A" (2004), Biosensors & Bioelectronics 20 (6), 1060-1067, discloses the use of a polymer derived from methacrylic acid, acrylamide, EGDMA, DMF and ochratoxin A for adsorbing ochratoxin A. The specificity of the polymer for ochratoxin A was found to vary under differing reaction conditions.

Sairam, M. et al, "Encapsulation efficiency and controlled release characteristics of crosslinked polyacrylamide particles" (2006), International Journal of Pharmaceutics 320 (1-2), 131-136, discloses the release characteristics of 5-fluorouracil from microparticles derived from polyacrylamide particles crosslinked with NNMBA or EGDMA and loaded with 5-fluorouracil. Release patterns were found to depend on the nature and amount of crosslinking agent and amount of 5-fluorouracil loaded.

An object of the present invention is to provide a polymer that can be used as an SPE adsorbent that is selective for aflatoxins.

### Summary of the Invention

The present invention is based on the finding that polymers containing amide, amine, pyridine, phosphate or sulfonate moieties are able to immobilise aflatoxins when used as an SPE adsorbent

Accordingly, the present invention provides the use of a polymer containing moieties derived from an acrylamide based monomer, prepared without use of a template, as an SPE adsorbent to immobilise aflatoxins.

Preferably the polymer is one containing moieties derived from an acrylamide based monomer, for example one or more of methylene bisacrylamide (MBBA), acrylamide or acrylamido-2-methylpropane-sulfonic acid (AMPSA), or a derivative thereof.

The polymers used in the invention may be prepared by conventional polymerisation techniques without use of aflatoxins, or structural analogues, as templates. Preferably the polymers are prepared in macroporous form, for example by use of a porogen during polymerisation.

A further aspect of the present invention is an SPE adsorbent unit comprising a column, cuvette, rod, needle, membrane or flat surface loaded or coated with an adsorbent layer of the above-mentioned polymers.

A further aspect of the present invention is a fluorometric analysis for aflatoxins which comprises adsorbing the aflatoxins the above-mentioned polymers loaded in or on a cuvette, cartridge, rod or flat surface, exposing the immobilised aflatoxins to UV light and detecting the fluorescence emitted by the immobilised aflatoxins.

Monomers which can be used for polymer preparation to provide appropriate moieties in the polymer include: allylamine; methylene bisacrylamide (MBAA); acrylamide, ethyleneglycolmethacrylate phosphate (EGMP); 4-vinyl pyridine; and 2-acrylamido-2-methylpropanesulfonic acid (AMPSA). Preferred embodiments are polymers containing acrylamide moieties such as acrylamide and MBAA, which are especially effective.

Preferably the functional monomer is cross-linked with an effective crosslinking agent that does not interfere with the interaction with aflatoxin. Suitable cross-linkers include polyol diacrylates, such as ethylene glycol dimethacrylate (EGDMA) and also divinyl benzene (DVB) and their analogues.

### Brief Description of the Drawings

Figure 1 is a cross-sectional view of an SPE adsorbent cartridge in accordance with the invention;
Figure 2 is a cross-sectional view of an alternative configuration of the SPE adsorbent cartridge of Figure 1.

### Detailed Description of the Invention

The polymer comprising a polymer unit containing amide, amine, pyridine, phosphate or sulfonate moieties that is the primary component of the present invention may be prepared by polymerising appropriate monomers containing amide, amine, pyridine, phosphate or sulfonate moieties (the functional monomers).

The polymer is preferably made porous to increase the surface area available for interaction with aflatoxins. The porous polymer may be prepared by polymerising a functional monomer and a cross-linker in the present of a porogen i.e. a material that is dispersible in the monomers, and remains dispersed in the polymers after reaction of the monomers, but can be removed after the polymer is formed to form pores within the polymer. Typically an increase in porosity leads to increase in surface area which could be used for maximising binding capacity.

Typically a suitable porogen is inert in the polymerisation reaction. Porogens may be solids, liquids or gases. Solids or liquids can be removed by decomposition or by dissolving out with a suitable solvent. Typically a liquid porogen is used that can be finely dispersed in the polymerisation mixture by stirring, and can be removed by washing the polymer with a suitable solvent.

When the functional monomers previously listed are cross-linked with the favoured cross-linkers ethylene glycol dimethacrylate (EGDMA) and divinyl benzene (DVB), then a suitable porogen is N,N-dimethylformamide (DMF). Acetonitrile, methanol, toluene, ethanol, glycerol or other solvents used in practice for radical polymerisation may also be used.

The reaction of the functional monomer with the selected cross-linking agent is carried out under conventional conditions suitable for the reactants. For example reaction of the listed functional monomers with the cross-linker ethylene glycol dimethacrylate and divinyl benzene may be carried out under elevated temperatures (i.e. above ambient), or under UV-irradiation at low temperatures (i.e. below ambient), or by precipitation polymerisation, in the presence of a suitable initiator, such as 1,1-azobis(cyclohexanecarbonitrile). Many different initiators of radical, atom transfer, ionic polymerizations may also be used as well for developing suitable polymers.

The monomers may also be used for formation of graft polymers on the surface of beads, membranes, surfaces or other articles using chemical, UV or plasma processes known by practitioners in this field.

In the SPE polymer the proportion of units derived from the functional monomer to the units derived from the cross-linker may vary from about 5:1 to about 1:100. Ratios with the range of about 1:3 to 1:20 has been found to be especially effective. For example, a ratio of 1:4 provides an effective absorbent in a polymer produced by thermal polymerisation, and a ratio of 1:19 in a polymer produced by UV-polymerisation at low temperature.

While EGDMA and divinyl benzene (DVB) are the favoured cross-linkers, other monomers capable of crosslinking the functional bisacrylamide polymer may be used. The cross-linker should preferably be of similar reactivity to the functional monomer. Suitable cross-linkers include, but are not limited to, ethylene glycol dimethacrylate (EGDMA), glycerol dimethacrylate (GDMA), trimethylacrylate (TRIM), divinylbenzene (DVB) and trimethylolpropane trimethacrylate.

Practitioners in this field will be able to select additional monomers and cross-linkers suitable for a particular system.

For analytical use in the immobilisation of aflatoxins, the finished polymer may be loaded into or onto or integrated with a suitable column, cuvette, needle, membrane, rod or flat surface for use as an SPE adsorbent unit. The support for the SPE adsorbent is preferably selected from a material that does not fluoresce in a way that will interfere with detection of fluorescence from the adsorbed aflatoxin, or which fluoresces at a manageable background level. The polymer is preferably loaded as a powder or monolith material which rests in a layer against a preformed frit as found in commercially available empty SPE cartridges, or as a uniform layer applied to a rod, cuvette, needle, membrane or flat surface.

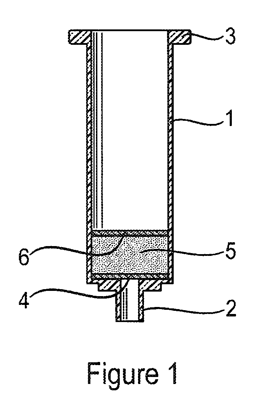

A typical unit is shown in Figure 1 of the accompanying drawings. An empty cylindrical SPE cartridge 1 (commercially available from, for example, Supelco of Poole, UK) has an outlet spout 2 and support flanges 3 at its inlet. The cartridge is made from plastics material that suitably has low fluorescence and that is ideally non-fluorescent under the conditions which stimulate fluorescence of aflatoxin. A porous frit 4, preferably of non-fluorescent material such as PTFE, is placed across the aperture giving access to the outlet spout 2. Adsorbent polymer in powder form is loaded into the cartridge to form a polymer layer 5. A further retaining frit 6 may be placed against the upper surface of the polymer layer 5. Alternatively a suitably shaped monolith of porous polymer is placed on top of the frit 4.

In use of the SPE unit, a liquid extract from a material potentially containing an aflatoxin is poured into the cartridge and allowed to drain (naturally or under suction) through the absorbent layer 5, passing out of the cartridge through the outlet spout 3. Any aflatoxin in the sample is absorbed by the polymer and forms a layer or band in the upper region of the polymer, below the frit 6, if present.

The thus-formed SPE adsorbent units may be used for quantitative analysis of adsorbed aflatoxins by inserting the unit into an analytical fluorimeter, for example, as described in WO 2006/123189, the entire disclosure of which is incorporated herein by reference.

To reduce background fluorescence during the analysis, it is useful to provide an opaque area or strip on the surface of the cartridge, to cover the polymer just below the region where the fluorescent band of adsorbed aflatoxin will appear. The effect may be supplemented by a further opaque area or strip on the cartridge just above the frit 6 or the region where the fluorescent band will appear.

The opaque areas may be formed by applying adhesive tape, such as masking tape or electric insulation tape, preferably black tape, to a cartridge after loading the polymer. Alternatively, the tape can be applied before loading the polymer, if the upper level of the polymer can be accurately predicted.

In a commercial process where the polymer is loaded in accurately controlled conditions, a cartridge may be used that has opaque areas formed during manufacture, for example, by coating or spraying with an opaquing material.

This technique for reducing background fluorescence is applicable to other SPE cartridges, or the previously mentioned mini-columns with mineral adsorbents, where a fluorescable band of adsorbed compounds is created in an absorbent material. Accordingly, this forms a further aspect of the invention independent of the above-described use with the polymer absorbents of this invention.

The SPE adsorbent units may also be used for the clean-up of samples prior to quantitative analysis by HPLC or ELISA.

The polymers may optionally be treated with a mineral oil to improve the binding and the fluorescent output of an aflatoxin band immobilised on the polymer (see Piletska E. V., Romero-Guerra M., Guerreiro A. R., Karim K., Turner A. P. F., Piletsky S. A. (2005) Adaptation of the molecular imprinted polymers towards polar environment. Anal. Chim. Acta, 542, 47-51.)

An SPE adsorbent made from a porous polymer of methylene bisacrylamide with the favoured cross-linker ethylene glycol dimethacrylate or divinyl benzene has been found to be especially effective to immobilise aflatoxins B₁, B₂, G₁ and G₂.

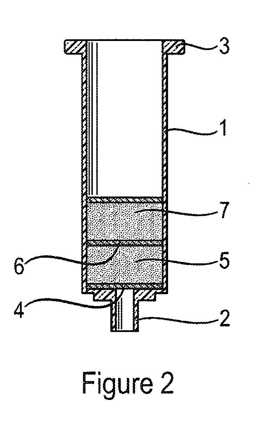

In another embodiment of the invention, shown in Figure 2, a further polymer layer 7 may be added to the cartridge on the frit 6 above the SPE adsorbent. This can be used to provide additional clean-up of sample solutions delivered to the cartridge, when required..

For aflatoxins, a suitable additional clean-up layer is provided by a diethyl aminoethyl-methacrylate (DEAEM) based polymer, which has been found to successfully remove potentially interfering compounds from samples taken from groundnuts (peanuts). Other suitable clean-up materials and procedures will be well known to those familiar with analysis for the presence of aflatoxins. Care must be taken to avoid diminishing the amount of aflatoxin while cleaning up a sample.

As well as the use for quantitative analysis described above the SPE polymer of this invention may find use as sheets or membranes to bind aflatoxins, or in sensors as disclosed in WO 2006/120381.

Also, it has been found that the aflatoxin may be easily removed from the polymer by washing with methanol and then with water. This may be used to 'regenerate' the polymer for further use, but it is not recommended to re-use the polymer when carrying out important qualitative or quantitative testing.

However it means that the polymer may be used as a clean-up step for samples which will be subjected to quantitative measurement in methods other than fluorometric analysis, such as chromatography. A liquid sample extract from a food product potentially containing aflatoxins is contacted with the polymer to adsorb any aflatoxins that are present, while other materials present in the sample are removed from the polymer by washing with a suitable solvent. Then the aflatoxins are eluted from the polymer and used as a clean solution for quantitative measurement of any aflatoxins present, for example by HPLC.

The implementation of the present invention will be further understood from the following Examples. (The intensity of the background fluorescence of the described polymers, and the fluorescent intensity of the immobilized aflatoxin, were determined by utilising a sensor device as disclosed in WO 2006/120381.)

### Example 1

### Preparation of Polymer

A polymerisation mixture was prepared by stirring the functional monomer methylene bisacrylamide (MBAA), 5g; a cross-linker ethylene glycol dimethacrylate (EGDMA), 20g, a porogen N,N-dimethylformamide (DMF), 25g; and an initiator 1,1-azobis(cyclohexanecarbonitrile), 500mg. The polymerisation mixture was illuminated for 20 min using a Hönle 100UV lamp (intensity 0.157 W/cm²) (Hönle UV, UK) followed by thermo-annealing in an oil bath at 80 °C for 12 hours. Washing with methanol removed the solvent (DMF) and the resulting polymer was a macroporous material. The resultant bulk polymers were ground and wet-sieved in methanol. The fraction with particle size in the range from 25 to 63 µm was collected and dried.

After preparation, 75mg of polymer were added to empty 1 ml non-fluorescent plastic cartridges (Supelco UK) fitted with non-fluorescent Teflon (PTFE) frits (Supelco UK)

### Example 2

### Immobilisation of AFB1 on MBAA-based polymer

Cartridges prepared as in Example 1 were pre-conditioned by addition of 2 ml water (e.g. HPLC grade water). Solutions of aflatoxin B1 (AFB1) in 4 ml of methanol (concentration of methanol varied from 80% to 20%, concentrations of AFB1 varying from 10 to 200 ng) were added to the cartridge to load the aflatoxins onto the layer of MBAA polymer. The cartridge was then washed to remove interfering compounds using 1 ml of 20% methanol (80:20, water:methanol). The presence of a layer of the aflatoxins immobilised in the layer of MBAA polymer was shown by observing the fluorescence generated by exposure to UV light, by utilising a sensor device as disclosed in WO 2006/120381.

A sharp fluorescent band was observed directly under the top frit of the SPE cartridge. The sensitivity of detection of AFB1 on MBAA-based polymer was very good, especially when adsorbed from 20% methanol.

### Example 3

### Immobilisation of Aflatoxins B₁, B₂, G₁, & G₂

Subsequently, 20% methanol solution was spiked with 100 ng of AFB1, AFB2, AFG1 and AFG2 toxins and loaded into individual cartridges, prepared as in Example 1, followed by a wash with 1 ml of 20% methanol. It was found that all toxins are adsorbed in very similar places, close to the top-frit.

### Example 4

### Testing of MBAA polymer in the maize and peanut matrices

A peanut matrix spiked with 20 ng of AFB1 was loaded on a cartridge prepared as in Example 1. The cartridge with loaded samples was washed with 1 ml of 20% methanol. The presence of a layer of AFB1 immobilised in the layer of MBAA polymer was shown by observing the fluorescence generated by exposure to UV light, by utilising a sensor device as disclosed in WO 2006/120381.

### Example 5

### Combination polymer cartridge for removal of interfering compounds

To a cartridge prepared as in Example 1 was added an upper polymer layer of a diethyl aminoethyl-methacrylate (DEAEM)-based polymer to provide a clean-up layer to immobilise any interfering compounds while allowing the aflatoxins to pass through to the MBAA-based polymer layer. The DEAEM-based polymer layer is effective to remove a significant proportion of the interfering components present in groundnuts (peanuts).

### Example 6

### Testing of AMPSA-based polymer on binding of AFB1

Using the procedure of Example 1, polymer cartridges were prepared with replacement of MBAA as the functional monomer by AMPSA. A polymer cartridge was washed with 2 ml of water and 4 ml of 80% methanol spiked with 200 ng of AFB1 was loaded into the cartridge. Next a fresh polymer cartridge was washed with 2 ml of water and 4 ml of 20% methanol spiked with 200 ng of AFB1 was loaded into the cartridge.

It was possible to see a band of AFB1 adsorbed on AMPSA-based polymer.

### Example 7

### Testing of 4-vinyl-pyridine-based polymer on binding of AFB1

Using the procedure of Example 1, polymer cartridges were prepared with replacement of MBAA as the functional monomer by 4-VP. A fresh polymer cartridge was washed with 2 ml of water and loaded with 80% methanol spiked with 200 ng of AFB1. The polymer cartridge was washed with 2 ml of water and loaded with 4 ml of 20% methanol spiked with 200 ng of AFB1.

4-VP-based polymer demonstrated binding of AFB1 from 20% methanol solution.

### Example 8

### Testing of allylanine-based polymer on binding of AFB1

Using the procedure of Example 1, polymer cartridges were prepared with replacement of MBAA as the functional monomer by allylamine. A polymer cartridge was washed with 2 ml of water and loaded with 4 ml of 80% methanol spiked with 200 ng of AFB1. A fresh polymer cartridge was washed with 2 ml of water and loaded with 4 ml of 20% methanol spiked with 200 ng of AFB1.

The allylamine-based polymer demonstrated some binding from both 80% and 20% methanol solutions.

### Example 9

### Testing of EGMP-based polymer on binding of AFB1

Using the procedure of Example 1, polymer cartridges were prepared with replacement of MBAA as the functional monomer by EGMP. A polymer cartridge was washed with 2 ml of water and loaded with 4 ml of 80% methanol spiked with 200 ng of AFB1. A fresh polymer cartridge was washed with 2 ml of water and loaded with 4 ml of 20% methanol spiked with 200 ng of AFB1.

EGMP-based polymer demonstrated binding of AFB1 from 20% methanol.

### Example 10

### Testing of acrylamide-based polymer on binding of AFB1

Acrylamide polymer demonstrated binding of AFB1 but the binding was weaker than for MBAA. The band was smaller and positioned in the middle of the layer.

### Example 11

### Mineral oil treatment

MBAA-based polymer cartridges were prepared as in Example 1. The polymer was treated with mineral oil by the following treatment:
- 1 ml of acetonitrile
- 1 ml of chloroform
- 2 ml of chloroform containing 1 % of mineral oil
- 1 ml of chloroform
- 1 ml of acetonitrile
- 2 ml of water

After mineral oil treatment, the MBAA cartridges showed an increase in fluorescence of AFB1 bound to the treated polymer.

### Example 12

### Regeneration of the MBAA-based cartridge

A cartridge of MBAA-based polymer prepared by Example 1 subjected to testing as in Example 2 was cleaned for re-use by treatment with:
- 4 ml of pure methanol
- 4 ml of water

This treatment was sufficient in order to completely remove the AFB1 adsorbed on the MBAA-based polymer and to prepare the cartridge for the next loading.

### Example 13

### Polymers prepared with DVB as a cross-linker

Cartridges were prepared as in Example 1 but using divinylbenzene (DVB) as cross-linker instead of with EGDMA. 100 ng of AFB1 in 20% methanol were loaded on the polymer.

It was found that the replacement of EGDMA with DVB did not cause any significant change in toxin adsorption on the cartridge

### Example 14

### Use of MBAA polymer for quantitative measurement of AFB1

Five cartridges were prepared as in Example 1 and increasing levels of AFB1 in 20% methanol were loaded on the polymer in order to afford cartridges loaded with fluorescent bands containing 0, 10, 20, 50 and 100 ng AFB1. The sensor device, as disclosed in WO 2006/120381, was used to measure the levels of immobilised toxin on each cartridge, by performing six measurements at each level. The mean readings at the various, increasing levels, were 0.73, 9.04, 20.24, 56.56 and 99.76ng, with corresponding co-efficients of variation of 4.41, 3.38, 2.64, 2.47 and 1.63%, respectively.

### Example 15

### UV polymerisation on ice (Polymer 1)

In order to investigate possible change in morphology of the polymer, polymerisation was performed using weak UV lamp (light intensity 0.015 kW). The polymerisation solution was kept at 4 °C (on ice). The polymerisation was conducted for 4 h. Because the solubility of MBAA in the DMF is lower at lower temperature the MBAA concentration in the polymer was reduced from 20% in the polymer prepared by thermo-polymerisation in Example 1 to 5% in the UV polymerisation. The polymerisation mixture was: 1 g of MBAA, 19 g of EGDMA (95% of cross-linking), 20 g of DMF, 200 mg of initiator 1,1-azobis (cyclohexanecarbonitrile).

The DMF was removed from the polymer by Soxhlet extraction with methanol (using approximately 100 cycles); and the polymer was then completely dried under vacuum. The resultant polymer was ground using Ultracentrifuge Mill (Retsch, UK) in order to get more regular uniform-sized particles. Several particle-size fractions were collected and the intensity of their background fluorescence and their ability to bind aflatoxin B₁ was evaluated. Among them were fractions with 25 - 63 µm, 63 - 125 µm and 125 - 212 µm particles.

It was found that all fractions had different background fluorescences with the lowest provided by fractions 63 -125 µm and 125 - 212 µm particles (Table 1). The 63 - 125 µm fraction produced the most intense fluorescence from the immobilised aflatoxin.

**Table 1. The properties and performance of the different fractions of the Polymer 1.**

| **Fraction** | **Background** | **Peak height** |
|---|---|---|
| 25 - 63 µm | 80,000 | 10 ng AFB1 - 45,000 |
| 63 - 125 µm | 75,000 | 10 ng AFB1 - 88,000 |
| 125 - 212 µm | 75,000 | 10 ng AFB1- 70,000 |

The properties of the polymer were compared with thermo-polymerised polymer which contained 20% of MBAA prepared in Example 1. It was found that the polymer produced by UV polymerisation had a significantly reduced background fluorescence. Although it had reduced quantity of the functional monomer (5% in comparison with 20% in case of thermo-polymer) it bound the aflatoxin quantitatively as a sharp band at the top of the cartridge.

The background and aflatoxin binding were also compared with a commercial SPE bonded phase adsorbent ("Phenyl", from Phenomenex). Although the background fluorescence of the "phenyl" adsorbent was significantly less than that of the MBAA polymer produced by UV polymerisation, the MBAA polymer strongly adsorbed the aflatoxin as a highly fluorescent band at the top of the polymer cartridge.

### Example 16

### MBAA-based polymer preparation with reduced quantity of solvent (DMF) and 5% of MBAA (Polymer 2)

In order to further change the polymer morphology a polymer was prepared which contained only half quantity of the solvent (DMF). All other conditions remained as in Example 15. The polymer was degassed, cooled down and kept on ice while polymerising with UV for 4 h. In order to dissolve 5 % of MBAA in the mixture with reduced solvent it was necessary to ultra-sonicate the mixture for 30 min.

**Table 2. The properties and performance of the different fractions of Polymer 2.**

| **Fraction** | **Background** | **Peak height** |
|---|---|---|
| 25 - 63 µm | 78,000 | 10 ng AFB1 - 30,000 |
| 63 - 125 µm | 80,000 | 10 ng AFB1 - 80,000 |
| 125 - 212 µm | > 100, 000 | - |

Fraction 63 -125 µm again had the optimal combination of background fluorescence and fluorescent intensity of the immobilised aflatoxin; and demonstrated similar characteristics to the corresponding fraction of Polymer 1.

### Example 17

### MBAA-based polymer preparation with doubled quantity of solvent (DMF) and 5 % MBAA (Polymer 3)

To make further change to the polymer morphology, a polymer was prepared using double the quantity of the solvent (DMF) relative to Example 15. All other conditions were as in Example 15. The polymer was degassed, cooled down and kept on ice while polymerising with UV for 4 h. The resulting polymer possessed a similar background intensity to Polymers 1 and 2 but afforded less than half the fluorescence from the immobilised aflatoxin.

### Example 18

### UV polymerisation at -20 °C, 1% MBAA (Polymer 4)

The impact of further decreasing the temperature during UV polymerisation was tested. Due to the reduced solubility of MBAA at -20 °C its concentration was reduced to 1%. All other parameters remained the same. The polymer was polymerised and processed as described earlier. Although the background was slightly less than Polymers 1 -3, prepared at +4 °C, Polymer 4 afforded a small fluorescent signal from the immobilised aflatoxin.

### Example 19

### MBAA-based polymer preparation with Tri(ethylene glycol) dimethacrylate cross-linker and 5 % MBAA (Polymer 5)

To change the polymer morphology again, Polymer 5 was produced using a different cross-linker (tri(ethylene glycol) dimethacrylate, 95%), and its performance compared with Polymer 1 which was produced using ethylene glycol dimethacrylate (EGDMA) as the cross-linker. The background fluorescence was very low and the fluorescence of the immobilised aflatoxin was reasonably high. However, the Polymer 5 was very flexible and changed its volume dramatically depending on the quantity of adsorbed solvent.

### Example 20

### The use of different solvents - DMSO (Polymer 6) and Toluene (Polymer 7)

Two more polymers (Polymers 6 and 7) were produced by UV polymerisation on ice for 4 h using different solvents (DMSO and toluene, respectively). The limited solubility of MBAA in these solvents permitted a concentration of only 1% of MBAA. All other parameters remained the same.. Polymers 6 and 7 exhibited a high background fluorescence and the immobilised aflatoxin produced a low fluorescence.

### Example 21

### Soxhlet washed Polymer 1

5g of Polymer 1 (5% MBAA); fraction 63 -125 µm) was washed in methanol in a Soxhlet extractor overnight (approximately 100 cycles of washing). The washing further improved the properties of the polymer by reducing the background fluorescence; and the fluorescence intensity of the immobilised aflatoxin was proportional to the quantity of adsorbed toxin..

### Example 22

### Binding 1 ng of AFB1, from 4 ml of 20% methanol, to washed Polymer 1

The binding of a low level of AFB1 from 4 ml of 20% methanol was evaluated.. When 4 ml of 20% methanol was spiked with 1 ng of AFB1 and loaded on the washed Polymer 1 (fraction 63 -125 µm), 1 ng of AFB1 was readily detected.

All these cartridges were also scanned quantitatively using a device of WO 2006/123,189. The results showed that the background fluorescence of the UV-polymer is almost half that of the thermo-polymer and about twice that of the "phenol" polymer.

An image of an SPE tube containing the UV-polymer with adsorbed 100 ng of aflatoxin B1 was also taken under UV. It was seen that the polymer had high affinity towards the aflatoxin and a band of aflatoxin was located just under the top-frit.

### Example 23

### Precipitation polymerisation

The polymerisation mixture was: 150 mg of MBAA, 600 mg of EGDMA (95% of cross-linking), 2 g of acetonitrile, and 30 mg of initiator (azobisisobutyronitrile (AIBN)). The reaction was conducted for 15 min. in 20 ml of mineral oil which was purged with a stream of nitrogen. The polymer mixture was also purged with nitrogen for 5 min and was transferred into the mineral oil. The mixture was dispersed using a homogeniser for 15 min, then illuminated with fiber optics UV lamp (Cermax, UK) for 2 h followed by illumination under stronger UV lamp (Honle, UK) for 20 min. The beads were filtered and washed with chloroform and acetone to remove the mineral oil. Although the polymer exhibited a reasonably low background fluorescence, and the immobilised aflatoxin produced a reasonably high fluorescence, the yield of polymer was low. The process also afforded a high quantity of waste mineral oil.

### Example 24

### Measurement of aflatoxins in food

The extraction of aflatoxins from food matrices (maize and peanut) using selected polymers was evaluated. 75 mg of the MBAA-based polymer of Example 15 was packed in the SPE tubes, and conditioned with 1 ml of 20% Methanol. The food extracts in 80% methanol were diluted four times with water and spiked with 10 ng of aflatoxin B1. As a control sample, food extract diluted with water four times was not spiked with toxin.

10 ng of aflatoxin was readily detected when immobilised on the MBAA-based polymer from either the maize or peanut extracts

### Example 25

### Modification of the optical characteristics of the polymer cartridge

When measured using a sensor device as disclosed in WO 2006/120381, the background fluorescence of the MBAA-based polymer, produced by thermo-polymerisation (Example 1), was significantly reduced by masking the emission of background fluorescence immediately above and below the band of immobilised toxin using thin strips of black masking tape.

10 ng of aflatoxin B₁ could readily be detected and measured using the optically modified cartridge.

## Claims

1. Use of a polymer containing moieties derived from an acrylamide based monomer as an SPE adsorbent for aflatoxins, wherein the polymer is prepared without use of aflatoxins, or structural analogues, as templates.

2. Use according to claim 1 in which the monomer is one or more of methylene bisacrylamide (MBBA), acrylamide or acrylamido-2-methylpropane-sulfonic acid (AMPSA), or a derivative thereof.

3. Use according to claim 1 or 2 in which the acrylamide based monomer has been cross-linked by copolymerisation with a cross-linking monomer.

4. Use according to claim 3 in which the cross-linker is ethylene glycol dimethacrylate (EGDMA) or divinyl benzene (DVB).

5. Use according to claims 3 or 4 in which the ratio of units of acrylamide monomer to crosslinker monomer is from 1:3 to 1:20.

6. Use according to any one of claims 1 to 5 in which the aflatoxins include one or more of aflatoxins B₁, B₂, G₁ and G₂.

7. Use according to any one of claims 1 to 6 in which the polymer and aflatoxin absorbed thereon is used as a sample in a fluorometric analysis method.

8. Use according to any one of claims 1 to 6 in which the adsorbed aflatoxin is eluted from the polymer and used as a sample in a quantitative measurement method.

9. Use according to any one of claims 1 to 8 in which the polymer has been obtained by UV-polymerisation at a temperature below ambient.

10. Use according to any one of claims 1 to 9 in which the polymer has been rendered macroporous by removal of a porogen present during polymerisation.

11. Use of a cross-linked polymer derived from methylene bisacrylamide (MBAA), allylamine, ethylene glycol methacrylate phosphate (EGMP), 4-vinyl pyridine or 2-acrylamido-2-methylpropane-sulfonic acid (AMPSA) as an SPE adsorbent for aflatoxins, wherein the polymer is prepared without use of aflatoxins, or structural analogues, as templates.

12. A fluorometric analysis method for aflatoxins which comprises: adsorbing the aflatoxins on a polymer derived from an acrylamide based monomer, prepared without use of aflatoxins, or structural analogues, as templates, the polymer being loaded in or on a cuvette, cartridge, needle, membrane, rod or flat surface; exposing the immobilised aflatoxins to UV light; and detecting the fluorescence emitted by the immobilised aflatoxin.

13. Method according to claim 12 in which the aflatoxins include one or more of aflatoxins B₁, B₂, G₁ and G₂.

14. A clean-up method prior to quantitative measurement of aflatoxins, in which a sample liquid is contacted with a polymer derived from an acrylamide based monomer prepared without use of aflatoxins, or structural analogues, as templates, the polymer being loaded in or on a cuvette, cartridge, rod, needle, membrane or flat surface to adsorb any aflatoxins that are present, and the aflatoxins are eluted from the polymer for quantitative measurement of any aflatoxins present.

15. An SPE adsorbent unit comprising a cuvette, cartridge, needle, membrane, rod or flat surface loaded or coated with a layer of methylene bisacrylamide (MBBA) cross-linked with ethylene glycol dimethacrylate (EGDMA), prepared without use of aflatoxins, or structural analogues, as templates, as polymer adsorbent.

## Patentansprüche

1. Verwendung eines Polymers, das Reste, die von einem Monomer auf Acrylamidbasis abgeleitet sind enthält, als ein SPE-Adsorbens für Aflatoxine, wobei das Polymer ohne Verwendung von Aflatoxinen, oder strukturellen Analoga, als Matrize hergestellt wird.

2. Verwendung nach Anspruch 1, bei welcher das Monomer eines oder mehrere von Methylenbisacrylamid (MBBA), Acrylamid oder Acrylamido-2-methylpropansulfonsäure (AMPSA) oder ein Derivat davon ist.

3. Verwendung nach Anspruch 1 oder 2, bei welcher das Monomer auf Acrylamidbasis durch Copolymerisation mit einem quervernetzenden Monomer quervernetzt worden ist.

4. Verwendung nach Anspruch 3, bei welcher der Quervernetzer Ethylenglycoldimethylacrylat (EGDMA) oder Divinylbenzol (DVB) ist.

5. Verwendung nach Anspruch 3 oder 4, bei welcher das Verhältnis der Einheiten des Acrylamidmonomers zum quervernetzenden Monomer von 1:3 bis 1:20 ist.

6. Verwendung nach einem der Ansprüche 1 bis 5, bei welcher die Aflatoxine eines oder mehrere der Aflatoxine B₁, B₂, G₁ und G₂ einschließen.

7. Verwendung nach einem der Ansprüche 1 bis 6, bei welcher das Polymer und darauf adsorbiertes Aflatoxin als eine Probe in einem fluorometrischen Analyseverfahren verwendet wird.

8. Verwendung nach einem der Ansprüche 1 bis 6, bei welcher das adsorbierte Aflatoxin von dem Polymer eluiert und als eine Probe in einem quantitativen Messverfahren verwendet wird.

9. Verwendung nach einem der Ansprüche 1 bis 8, bei welcher das Polymer durch UV-Polymerisation bei einer Temperatur unterhalb der Umgebungstemperatur erhalten wurde.

10. Verwendung nach einem der Ansprüche 1 bis 9, bei welcher das Polymer durch Entfernen eines während der Polymerisation vorhandenen Porogens makroproös gemacht wird.

11. Verwendung eines quervernetzten Polymers abgeleitet aus Methylenbisacrylamid (MBAA), Allylamin, Ethylenglycolmethacrylatphosphat (EGMP), 4-Vinylpyridin oder 2-Acrylamido-2-methylpropansulfonsäure (AMPSA) als ein SPE-Adsorbens für Aflatoxine, wobei das Polymer ohne Verwendung von Aflatoxinen, oder strukturellen Analoga, als Matrizen hergestellt wird.

12. Fluorometrisches Analyseverfahren für Aflatoxine, welches umfasst:
Adsorbieren der Aflatoxine auf einem Polymer, das aus einem Monomer auf Acrylamidbasis abgeleitet ist, das ohne Verwendung von Aflatoxinen, oder strukturellen Analoga, als Matrizen hergestellt wird, wobei das Polymer in oder auf eine Küvette, Kartusche, Nadel, Membran, Stab oder eine flache Oberfläche geladen wird; Exponieren der immobilisierten Aflatoxine mit UV-Licht; und Nachweisen der Fluoreszenz, die durch das immobilisierte Aflatoxin emittiert wird.

13. Verfahren nach Anspruch 12, in welchem die Aflatoxine eines oder mehrere der Aflatoxine B₁, B₂, G₁ und G₂ einschließen.

14. Reinigungsverfahren vor der quantitativen Messung von Aflatoxinen, in welchem eine Probenflüssigkeit mit einem Polymer, das von einem Monomer auf Acrylamidbasis abgeleitet ist, das ohne Verwendung von Aflatoxinen, oder strukturellen Analoga, als Matrizen hergestellt wird, in Kontakt gebracht wird, wobei das Polymer in oder auf eine Küvette, Kartusche, Stab, Nadel, Membran oder flache Oberfläche geladen wird, um alle vorhandenen Aflatoxine zu adsorbieren, und die Aflatoxine werden von dem Polymer für die quantitative Messung aller vorhandenen Aflatoxine eluiert.

15. Eine SPE-Adsorbenseinheit umfassend eine Küvette, Kartusche, Nadel, Membran, Stab oder flache Oberfläche, beladen oder beschichtet mit einer Schicht von Methylenbisacrylamid (MBBA), quervernetzt mit Ethylenglycoldimethylacrylat (EGDMA), das ohne Verwendung von Aflatoxinen, oder strukturellen Analoga, als Matrizen hergestellt wird, als Polymeradsorbens.

## Revendications

1. Utilisation d'un polymère contenant des fragments dérivés d'un monomère à base d'acrylamide comme adsorbant d'extraction en phase solide, dite SPE, pour les aflatoxines, où le polymère est préparé sans utiliser d'aflatoxines, ou d'analogues structuraux, en tant que matrices.

2. Utilisation selon la revendication 1 dans laquelle le monomère est l'un, ou plusieurs, parmi : le méthylène bisacrylamide (MBBA), l'acrylamide ou l'acide acrylamido-2-méthylpropane-sulfonique (AMPSA), ou l'un de leurs dérivés.

3. Utilisation selon la revendication 1 ou 2 dans laquelle le monomère à base d'acrylamide a été réticulé par copolymérisation avec un monomère réticulant.

4. Utilisation selon la revendication 3 dans laquelle le réticulant est le diméthacrylate d'éthylène glycol (EGDMA) ou le divinylbenzène (DVB).

5. Utilisation selon les revendications 3 ou 4 dans laquelle le rapport des unités du monomère d'acrylamide sur le monomère réticulant est de 1:3 à 1:20.

6. Utilisation selon l'une quelconque des revendications 1 à 5 dans laquelle les aflatoxines comportent une ou plusieurs aflatoxines des aflatoxines B₁, B₂, G₁ et G₂.

7. Utilisation selon l'une quelconque des revendications 1 à 6 dans laquelle le polymère et une aflatoxine absorbée sur celui-ci est utilisé comme échantillon dans un procédé d'analyse fluorimétrique.

8. Utilisation selon l'une quelconque des revendications 1 à 6 dans laquelle l'aflatoxine adsorbée est éluée à partir du polymère et utilisée comme échantillon dans un procédé de mesure quantitative.

9. Utilisation selon l'une quelconque des revendications 1 à 8 dans laquelle le polymère a été obtenu au moyen d'une polymérisation par UV à une température inférieure à la température ambiante.

10. Utilisation selon l'une quelconque des revendications 1 à 9 dans laquelle le polymère a été rendu macroporeux par élimination d'un agent porogène présent pendant la polymérisation.

11. Utilisation d'un polymère réticulé dérivé du méthylène bisacrylamide (MBAA), allylamine, phosphate de méthacrylate d'éthylène glycol (EGMP), 4-vinyl pyridine ou acide 2-acrylamido-2-méthylpropane-sulfonique (AMPSA) comme adsorbant d'extraction en phase solide, dite SPE, pour les aflatoxines, où le polymère est préparé sans utiliser d'aflatoxines, ou d'analogues structuraux, en tant que matrices.

12. Procédé d'analyse fluorimétrique pour les aflatoxines qui comprend le fait : d'adsorber les aflatoxines sur un polymère dérivé d'un monomère à base d'acrylamide, préparé sans utilisation d'aflatoxines, ou d'analogues structuraux, en tant que matrices, le polymère étant chargé dans ou sur une cuvette, une cartouche, une aiguille, une membrane, une tige ou une surface plane ; d'exposer les aflatoxines immobilisées à la lumière UV ; et de détecter la fluorescence émise par l'aflatoxine immobilisée.

13. Procédé selon la revendication 12 dans lequel les aflatoxines comportent une ou plusieurs aflatoxines des aflatoxines B₁, B₂, G₁ et G₂.

14. Procédé de nettoyage avant la mesure quantitative d'aflatoxines, dans lequel un échantillon liquide est mis en contact avec un polymère dérivé d'un monomère à base d'acrylamide préparé sans utilisation d'aflatoxines, ou d'analogues structuraux, en tant que matrices, le polymère étant chargé dans ou sur une cuvette, une cartouche, une tige, une aiguille, une membrane ou une surface plane pour adsorber toutes les aflatoxines qui sont présentes, et les aflatoxines sont éluées à partir du polymère pour la mesure quantitative de toutes les aflatoxines présentes.

15. Unité d'adsorbant pour l'extraction en phase solide comprenant une cuvette, une cartouche, une aiguille, une membrane, une tige ou une surface plane chargée ou revêtue d'une couche de méthylène bisacrylamide (MBBA) réticulée avec du diméthacrylate d'éthylène glycol (EGDMA), préparée sans utilisation d'aflatoxines, ou d'analogues structuraux, en tant que matrices, en tant que adsorbant polymère.
